# FASCICULE DE BREVET EUROPEEN

(11) **EP 0 975 791 B1**
(45) Date de publication et mention de la délivrance du brevet: **12.10.2005**
(21) Numéro de dépôt: 98912545.5
(22) Date de dépôt: 25.02.1998
(51) Int. Cl.: C12P 19/04, C12N 1/20

(54) **SOUCHE BACTERIENNE MARINE DU GENRE VIBRIO, POLYSACCHARIDES HYDROSOLUBLES PRODUITS PAR CETTE SOUCHE, ET LEURS UTILISATIONS**
MEERES-BAKTERIENSTAMM DER GATTUNG VIBRIO, DAVON HERGESTELLTE WASSERLÖSLICHE POLYSACCHARIDE UND DEREN VERWENDUNG
MARINE BACTERIAL STRAIN OF THE GENUS VIBRIO, WATER-SOLUBLE POLYSACCHARIDES PRODUCED BY SAID STRAIN AND THEIR USES

(30) Priorité: 25.02.1997 FR 9702199
(43) Date de publication de la demande: 02.02.2000
(73) Titulaire: IFREMER INSTITUT FRANCAIS DE RECHERCHE POUR L'EXPLOITATION DE LA MER, 92138 Issy-les-Moulineaux Cédex (FR)
(72) Inventeur: GUEZENNEC, Jean, F-29280 Plouzane (FR); PIGNET, Patricia, F-29200 Brest (FR); RAGUENES, Gérard, F-29200 Brest (FR); ROUGEAUX, Hélène, F-29200 Brest (FR)
(74) Mandataire: Nargolwalla, Cyra
(86) Numéro de dépôt international: PCT/FR1998/000368
(87) Numéro de publication internationale: WO 1998/038327

(56) Documents cités:
- WO-A-94/18340
- US-A- 5 393 777
- DATABASE WPI Week 9111 31 janvier 1991 Derwent Publications Ltd., London, GB; AN 91-076876 XP002048324 & JP 03 022 994 A (SAGAMI CHEM RES CENTRE) , 31 janvier 1991
- DATABASE WPI Week 7750 20 octobre 1975 Derwent Publications Ltd., London, GB; AN 77-88667Y XP002048325 & JP 50 132 189 A (MITSUBISHI KASEI CORP) , 20 octobre 1975
- DATABASE WPI Week 8504 11 décembre 1984 Derwent Publications Ltd., London, GB; AN 85-023383 XP002048326 & JP 59 220 197 A (SNOW BRAND MILK PRO CO LTD) , 11 décembre 1984
- G. RAGUENES ET AL.,: "Vibrio diabolicus sp. nov., a new polysaccharide-secreting organism isolated form a deep-sea hydrothermal vent polychaete annelid, Alvinella pompejana" INTERNATIONAL JOURNAL OF SYSTEMATIC BACTERIOLOGY, vol. 47, no. 4, - 1 octobre 1997 WASHINGTON DC, US, pages 989-995, XP002048323

## Description

La présente invention est relative à une nouvelle souche bactérienne du genre *Vibrio*, aux exopolysaccharides produits par ladite souche, et à leurs utilisations.

Certains micro-organismes issus du milieu hydrothermal sous-marin profond produisent des biomolécules dont la structure et la composition particulières leur conférent des propriétés d'un grand intérêt industriel potentiel ; parmi ces biomolécules, on trouve une grande variété de polysaccharides, dont certains ont déjà fait l'objet d'études visant à déterminer leur structures et leurs propriétés.

Les études citées ci-après ont concerné plus spécifiquement les exopolysaccharides (EPS) excrétés par des bactéries du genre *Alteromonas* cultivées en conditions de laboratoire, et en particulier sur milieu enrichi en glucose.

VINCENT et al. [Appl. Environ. Microbiol., 60, 4134-4141 (1994)] ont ainsi décrit un exopolysaccharide, dénommé EPS-1545, excrété par une souche, désignée sous la référence HYD-1545, d'une bactérie du genre *Alteromonas*. L'EPS obtenu par précipitation à l'éthanol comprend entre 49 et 55% d'oses neutres, et entre 32,5 et 39% d'acides uroniques.

GUEZENNEC et al. [Carbohydrate Polymers, 24, 287-294 (1994)] ont classé les EPS produits par différentes bactéries du genre *Alteromonas* en 5 groupes différents sur la base de leur composition : le groupe 1 est constitué d'EPS comprenant entre 50 et 60% environ d'oses neutres, environ 10% d'acides uroniques, entre 0,5 et 3,7% d'osamines, et entre 11,5 et 21,5% de sulfates ; le groupe 2 est constitué d'EPS comprenant environ 50% d'oses neutres, possédant une faible teneur en acides uroniques (8%), comprenant entre 1 et 3,2% d'osamines, et dont la teneur en sulfates est comprise entre moins de 10% et 13% ; les EPS du groupe 3 comprennent entre 40 et 50% d'oses neutres, ont une très faible teneur en acides uroniques (entre 5 et 7%), comprennent entre 1,2 et 1,7% d'osamines, et ont une teneur en sulfates variant entre 8,9 et 17,2 ; le groupe 4 est constitué d'EPS comprenant 46 à 49% d'oses neutres, possédant une teneur élevée en acides uroniques (entre 34 et 40%), comprenant entre 0,2 et 1,6% d'osamines, et ayant une teneur en sulfates comprise entre 9,7 et 13% ; le groupe 5 est constitué d'EPS à teneur relativement faible en oses neutres (38 à 47%), à teneur élevée en acides uroniques (26 à 32%), comprenant entre 1 et 1,6% d'osamines, et entre 5,2 et 10,1% de sulfates ; l'un des EPS de ce groupe comprend en outre un acide hexuronique portant un substituant lactate.

Un certain nombre des souches et des polymères décrits dans les publications de VINCENT et al. et GUEZENNEC et al. citées ci-dessus font l'objet de la demande PCT FR 94/00169, publiée sous le numéro WO/9418340.

Récemment, RAGUÉNÈS et al. [Appl. Environ. Microbiol. 62, 67-73 (1996)] ont décrit un EPS, différent des précédents, obtenu à partir d'une bactérie du genre *Alteromonas,* (*Alteromonas macleodii* subsp. *fijiensis*). Cet EPS comprend environ 38% d'oses neutres, 38% d'acides uroniques, 2,3% d'osamines, et environ 5% de sulfates.

Les Inventeurs, en poursuivant leurs recherches sur les bactéries issues du milieu hydrothermal sous-marin profond ont maintenant isolé à partir d'un ver de Pompéi (*Alvinella pompejana*) une nouvelle souche, dénommée HE800, appartenant au genre *Vibrio.* Cette souche, qui a été déposée le 17 Octobre 1995 auprès de la CNCM (Collection Nationale de Cultures de Microorganismes, 28, rue du Docteur Roux, 75724 PARIS, FRANCE) sous le numéro I-1629, représente une nouvelle espèce de *Vibrio,* pour laquelle l'appellation *Vibrio diabolicus* est proposée.

*Vibrio diabolicus* est un bacille gram négatif, d'environ 0,8 µm de large et 2,2 µm de long ; il est mobile à l'aide d'un flagelle polaire en milieu liquide et de flagelles péritriches en milieu solide ; c'est une bactérie non-encapsulée, non-pigmentée, et non-luminescente.

C'est une bactérie anaérobie facultative, fermentative, catalase +, cytochrome oxydase +, chitinase +. Elle réduit les nitrates en nitrites. Elle est sensible à l'agent vibriostatique 0/129 (2,4 diamino-6-7-diisopropylptéridine).

Elle utilise une large variété de substrats carbonés ; elle peut utiliser en particulier comme seule source de carbone l'un quelconque des substrats suivants : glycérol, ribose, galactose, glucose, fructose, mannose, mannitol, N-acétyl glucosamine, maltose, sucrose, tréhalose, amidon, glycogène, gluconate, caprate, citrate, et malate.

Sa croissance est optimale à une température comprise entre 30 et 45°C, un pH compris entre 7 et 8, et une salinité comprise entre 20 et 50 g/l de NaCl ; son temps de génération dans ces conditions est compris entre 18 et 28 minutes.

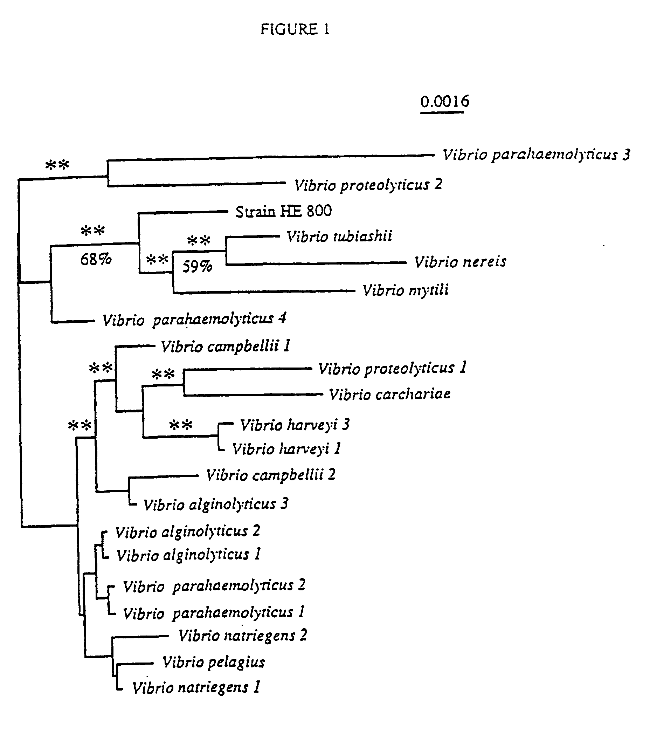

La teneur en G+C de son ADN est de 49,6%. L'analyse phylogénique du gène 16S rRNA (selon le protocole établi par RUIMY et al. [Int. J. Syst.Bacteriol 44, 416-426 (1994)], a permis d'établir qu'elle appartient à un taxon bien défini qui inclut également *Vibrio mytili, Vibrio nereis,* et *Vibrio tubiashii*. Les résultats de cette analyse sont illustrés par la Figure 1. Le pourcentage d'homologie de l'ADN de la souche HE800 avec celui des 3 *Vibrio* les plus proches mentionnées ci-dessus est respectivement de 27%, 15%, et 5%.

Le tableau I ci-après illustre les propriétés métaboliques de la souche HE800 (Seules les réponses positives sont incluses dans ce tableau)

**TABLEAU I**

| | |
|---|---|
| Utilisation du glycérol | + |
| Utilisation du ribose | + |
| Utilisation du galactose | + |
| Utilisation du glucose | + |
| Utilisation du fructose | + |
| Utilisation du mannose | + |
| Utilisation du mannitol | + |
| Utilisation de la N-acétyl-glucosamine | + |
| Utilisation du maltose | + |
| Utilisation du sucrose | + |
| Utilisation du tréhalose | + |
| Utilisation de l'amidon | + |
| Utilisation du glycogène | + |
| Utilisation du gluconate | + |
| Utilisation du caprate | + |
| Utilisation du citrate | + |
| Utilisation du malate | + |
| Réduction des nitrates en nitrites | + |
| Production d'indole | + |
| Acidification du glucose | + |
| Hydrolyse de l'esculine (β-glucosidase) | + |
| Hydrolyse de la gélatine (protéase) | + |
| b-galactosidase (PNPG) | + |
| Lysine décarboxylase | + |
| Ornithine décarboxylase | + |
| Tryptophane désaminase | + |
| Phosphatase alcaline | ++ |
| Estérase (C4) | + |
| Estérase lipase (C8) | +++ |
| Leucine arylamidase | +++ |
| Trypsine | + |
| Chymotrypsine | + |
| Phosphatase acide | +++ |

Les caractéristiques morphologiques, biochimiques et phylogénétiques ci-dessus permettent d'inclure la souche HE800 dans le genre *Vibrio* [BAUMANN et al. : Genus *Vibrio*, Bergey's Manual of Systematic Bacteriology, Vol. 1, 342-352. (1984) KRIEG, and HOIT (ed.) ; The Williams and Wilkins Co., Baltimore] ;

Du fait que le pourcentage d'homologie ADN/ADN avec les trois sous-espèces du genre *Vibrio* mentionnées ci-dessus est inférieur à 30%, la souche HE800 peut être considérée comme représentant une nouvelle espèce de *Vibrio* [WAYNE et al., Report of the Ad Hoc Commitee on reconciliation of approaches to bacterial systematics, Int. J. Syst. Bacteriol. 463-464, (1987)], pour laquelle l'appellation : *Vibrio diabolicus* est proposée.

La présente Invention a pour objet des souches de bactéries possédant les caractéristiques, définies ci-dessus, de l'espèce *Vibrio diabolicus*, et en particulier de la souche HE800 ; ceci englobe en particulier les bactéries dont l'ADN a un pourcentage d'homologie supérieur à 30%, et avantageusement supérieur à 50%, avec l'ADN de la souche HE800. Cet objet englobe également les bactéries obtenues à partir de bactéries de l'espèce *Vibrio diabolicus*, et en particulier de la souche HE800, par mutation, ou par recombinaison génétique, telles que par exemple des bactéries de l'espèce *Vibrio diabolicus* hébergeant un plasmide portant un gène hétérologue.

La présente Invention englobe également les différents produits qui peuvent être obtenus à partir desdites bactéries, ce qui comprend notamment des polysaccharides susceptibles d'être obtenus à partir des surnageants de cultures de Vibrio *diabolicus*, et en particulier un exopolysaccharide susceptible d'être obtenu par précipitation à l'éthanol, à partir de surnageants de cultures de la souche HE800.

L'analyse faite par les Inventeurs d'un exopolysaccharide conforme à la présente invention, produit par la souche HE800, fait apparaître les caractéristiques suivantes, déterminées à partir d'une préparation dudit polysaccharide comprenant environ 1% en poids de protéines :
- il ne comprend pas d'oses neutres ;
- sa teneur en osamines est d'environ 30 ± 5% en poids
- sa teneur en acides uroniques est d'environ 32 ± 5% en poids ;
- sa composition en monosaccharides, déterminée après méthanolyse acide, est la suivante : environ 11,2% en poids d'acide glucuronique, environ 18% en poids de N-acétyl glucosamine, environ 7,9% en poids de N-acétyl galactosamine, soit 1,4 moles de N-acétyl glucosamine et 0,6 moles de N-acétyl galactosamine pour 1 mole d'acide glucuronique ;
- il ne comprend pas d'unités saccharidiques sulfatées ;
- sa viscosité intrinsèque est de l'ordre de 570 ml/g ;
- son poids moléculaire moyen est de l'ordre de 800 000 Da.

Ce polysaccharide possède une composition voisine de celle de l'héparine, qui est couramment utilisée en industrie pharmaceutique, en particulier pour ses propriétés anticoagulantes et anti-thrombotiques. Il en diffère toutefois en particulier par la présence de N-acétyl galactosamine et par l'absence de groupements sulfate.

Du fait de sa structure, ce polysaccharide est utilisable en particulier dans le cadre de l'industrie pharmaceutique, par exemple en tant que matière première dans le cadre de l'obtention de médicaments. Dans ce but, il peut par exemple être modifié, notamment par voie chimique ou enzymatique pour obtenir des fractions sulfatées, et/ou de faible poids moléculaire, utilisables par exemple comme agents anti-viraux, antitumoraux, ou anti-thrombotiques.

La présente Invention sera mieux comprise à l'aide du complément de description qui va suivre; qui se réfère à des exemples décrivant la préparation et la caractérisation de polysaccharides conformes à l'Invention.

Il va de soi toutefois que ces exemples sont donnés uniquement à titre d'illustration de l'objet de l'Invention, dont ils ne constituent en aucune manière une limitation.

### EXEMPLE 1 : PRÉPARATION D'EXOPOLYSACCHARIDE À PARTIR DE CULTURES DE VIBRIO DIABOLICUS

### a) Cultures de Vibrio diabolicus

La souche HE800 est cultivée sur milieu 2216E [OPPENHEIMER, J. Mar. Res. 11, 10-18 (1952)] enrichi avec du glucose (30 g/l). La production est effectuée à 30°C et à pH 7,4 en fermenteur de 2 litres contenant 1 litre du milieu 2216E-glucosé. Après 48 heures de culture, le moût présente une faible viscosité (de l'ordre de 40 centipoises à 60rpm).

### b) Purification de l'exopolysaccharide

Les bactéries sont séparées du moût par une centrifugation à 20 000 g pendant 2 heures, puis le polysaccharide est précipité à partir du surnageant à l'aide d'éthanol pur, puis on effectue plusieurs lavages éthanol/eau en proportions croissantes d'éthanol, selon le procédé décrit par TALMONT et al. [Food Hydrocolloids 5, 171-172 (1991)] ou VINCENT et al. [Appl. Environ. Microbiol., 60, 4134-4141 (1994)]. Le polymère obtenu est séché à 30°C et conservé à température ambiante. 2,5 g de polysaccharide purifié par litre de culture ont ainsi été obtenus.

### EXEMPLE 2 : CARACTERISATION PHYSICO-CHIMIQUE DE L'EXOPOLYSACCHARIDE PRODUIT PAR LA BACTERIE VIBRIO DIABOLICUS.

### a) Analyse chimique :

Les teneurs en oses neutres, oses acides, oses amines et protéines du polysaccharide obtenu comme décrit à l'exemple 1 ci-dessus, sont déterminées selon les méthodes colorimétriques suivantes:

### Dosages des oses neutres

Méthode de TILLMAMNS et PHILLIPPI [Biochem. J., 215, 36-60, (1929)] modifiée par REMINGTON [Biochem. J., 25, 1062-1071, (1931)].
Modification apportée: Pour éliminer l'interférence due aux acides uroniques, ceux-ci sont dosés par la méthode de DISCHE [J. Biol Chem. 167, 189-198, (1947) ; MONTREUIL J. et SPIK G., Méthodes colorimétriques de dosage des glucides totaux - Microdosage des glucides, Fascicule 1. (1963)].
Réactif: Orcinol sulfurique
Référence: 1 mannose/l galactose

### Dosages des oses acides:

Deux méthodes ont été utilisées
1) Méthode de DISCHE (1947 ;référence citée ci-dessus).
   Modification apportée : Pour éliminer l'interférence due aux hexoses ceux-ci sont dosés, comme mentionné ci-dessus, par la méthode de TILLMANS et PHILLIPPI (1929) modifiée par REMINGTON (1931).
   Réactif: Carbazol
   Référence: Acide glucuronique
2) Méthode de BLUMENKRANTZ et ASBOE-HANSEN [Anal. Biochem. 54, 484-489, (1973)].
   Réactifs: Méta-hydroxy diphényl

Tétraborate de sodium 0,0125 M dans acide sulfurique concentré
Référence : Acide glucuronique.

### Dosage des osamines:

Méthode D'ELSON et MORGAN [Biochem. J. 27, 1824-1828(1933)] modifiée par BELCHER et al. (Modification de la composition du réactif d'EHRLICH).
Modification apportée: Transformation du réactif d'EHRLICH selon le procédé de BLIX (MONTREUIL et SPIK,. Microdosage des glucides, Fascicule 1, 1963).
- Réactifs:: solution alcaline d'acétyl-acétone
Réactif d'Ehrlich
- Référence:: glucosamine.

### Dosage des protéines

Méthode de LOWRY [LOWRY et al. [J. Biol. Chem. 193, 265-275, (1953)].
- Réactifs:: Réactif A
Réactif B
- Réactif FOLIN-CIOCALTEU :: 1N
- Référence :: albumine bovine.

### Composition en monosaccharides.

Le polysaccharide est soumis à une méthanolyse acide afin de les mettre sous la forme de méthylglycosides N-acétylés et 0-triméthylsilylés selon la méthode de KAMERLING et al, [Biochem. J., 151, 491-495,(1975)], modifiée par MONTREUIL [MONTREUIL et al., Glycoproteins, *in* Carbohydrate Analysis : A practical approach : Eds CHAPLIN et KENNEDY, IRL Press, Oxford, Washington DC, p. 143-204, (1986)].

Les différents sucres obtenus sont alors analysés par chromatographie en phase gazeuse, le cas échéant par couplage chromatographie phase gazeuse/spectrométrie de masse.
Ces analyses montrent la présence d'acide glucuronique, de N-acétyl glucosamine, et de N-acétyl galactosamine, dans des proportions en poids de 11,2%, 18%, et 7,9% respectivement. On détecte également de l'acide galacturonique en faible quantité (0,5% en poids). Les oses neutres (glucose et mannose) ne sont détectés qu'à l'état de traces, reflétant probablement une contamination.

### b) Analyse spectroscopique : RMN et Spectroscopie Infrarouge

La présence de groupes acétate et carboxyliques a été recherchée par Résonance Magnétique Nucléaire (RMN) et Spectroscopie Infrarouge à Transformée de Fourier. Cette dernière méthode permet en outre de quantifier la teneur éventuelle en sulfates (LIJOUR et al., 1994, Anal. Biochem., 220, 244-248).

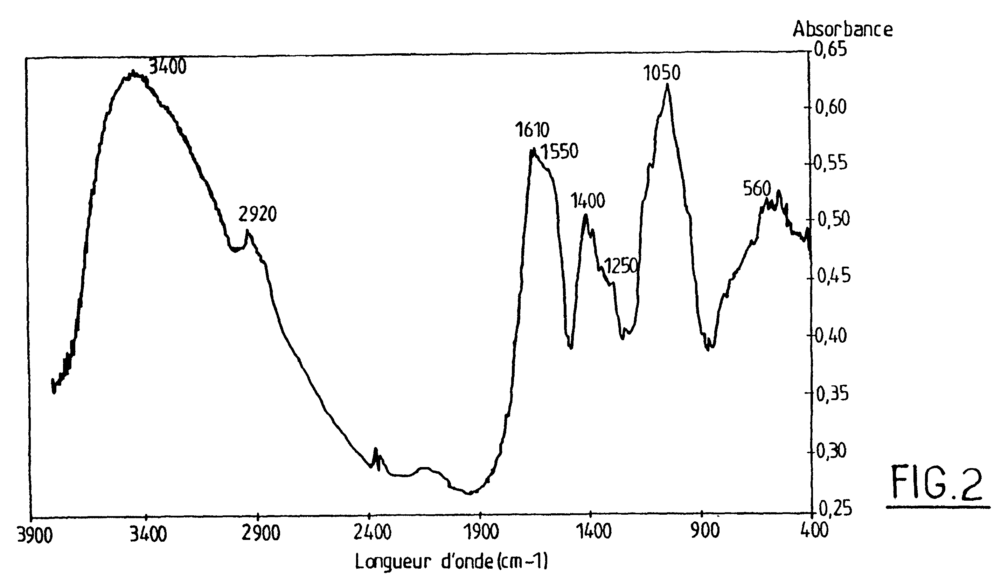

Le spectre infra-rouge est représenté sur la figure 2.

Ce spectre permet de mettre en évidence des groupements carboxylés et N-acétylés, ce qui confirme la présence d'acides uroniques. On constate en outre l'absence de groupements sulfate.

Les analyses de RMN (¹³C et ¹H) confirment également la présence des constituants mis en évidence par l'analyse chromatographique, et l'absence de substituants tels que le pyruvate ou le succinate.

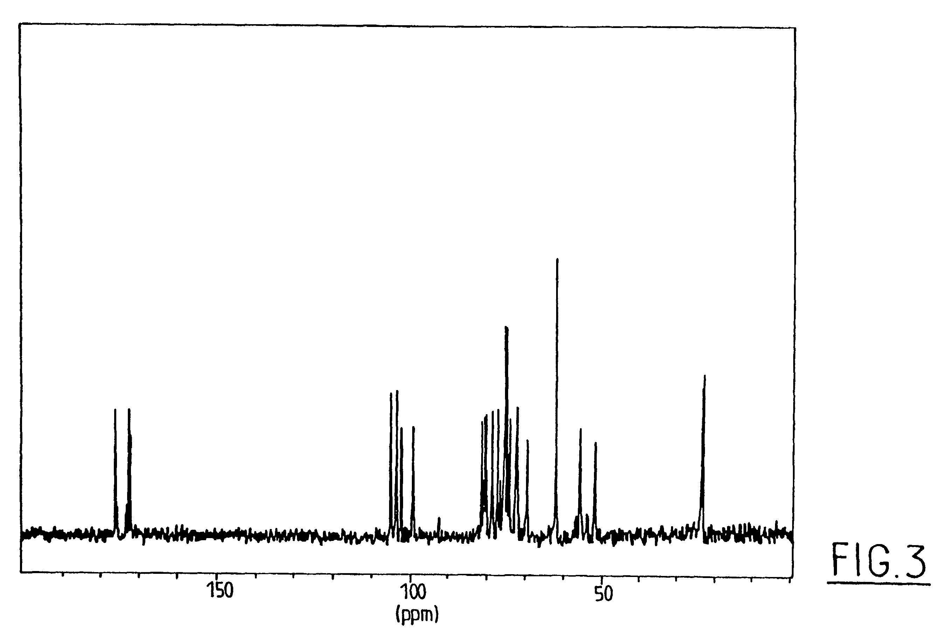

Le spectre de RMN ¹³C, représenté sur la Figure 3 met en évidence 4 carbones anomériques (100.3, 103.7, 104.9, et 106.3 ppm) ainsi que 4 carbones carboxyliques (173.6, 174.2, 177.0, et 177.3 ppm). Des signaux à 25.0 ppm et 25.5 ppm peuvent être attribués à des groupes méthyle ou acétyle, indiquant la N-acétylation des hexosamines.

### c) Etudes de rhéologie.

La viscosité intrinsèque [η] (volume hydrodynamique occupé par un gramme de polymère dans un solvant donné) de la préparation de polysaccharides obtenus comme décrit dans l'exemple 1, en solution dans l'eau distillée additionnée de NaCl à une concentration de 0,1 M, a été déterminée, par l'établissement de courbes d'écoulement en régime dilué à différentes concentrations.

L'étude des propriétés d'écoulement de ce polymère a également été réalisée en régime semi-dilué sur une solution à 0,3% de polysaccharide. Elle se traduit par une courbe d'écoulement donnant la viscosité relative ηr en fonction du taux de cisaillement, la solution ayant subi un cycle d'augmentation et de diminution de vitesse en continu.

L'ensemble des mesures a été effectué à 20°C en utilisant un viscosimètre à cylindres co-axiaux LOW SHEAR 40 (CONTRAVES).

La viscosité intrinsèque a ainsi été évaluée à 570 ml/g .

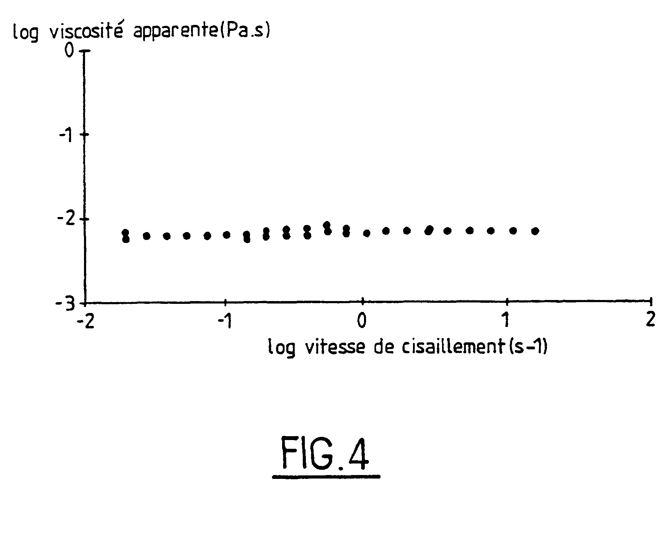

Les courbes d'écoulement de ce polysaccharide en solution à 0,3% dans NaCl 0,1M sont représentées sur la Figure 4.

## Revendications

1. Souche bactérienne du genre *Vibrio*, **caractérisée en ce qu'**elle appartient à l'espèce dénommée *Vibrio diabolicus*, et possède les caractéristiques définies ci-dessous ;
- bacille gram négatif droit, d'environ 0,8 µm de large et 2,2 µm de long ;
- mobile à l'aide d'un flagelle polaire en milieu liquide et de flagelles péritriches en milieu solide ;
- non-encapsulée, non-pigmentée, et non-luminescente ;
- anaérobie facultative, catalase +, fermentative, cytochrome oxydase +, chitinase +, réduisant les nitrates en nitrites, et sensible à la 2,4 diamino-6-7-diisopropylptéridine ;
- pouvant utiliser comme seule source de carbone l'un quelconque des substrats suivants : glycérol, ribose, galactose, glucose, fructose, mannose, mannitol, N-acétyl glucosamine, maltose, sucrose, tréhalose, amidon, glycogène, gluconate, caprate, citrate, malate ;
- ADN ayant une teneur en G+C de 49,6%, un pourcentage d'homologie de 27% avec l'ADN de *Vibrio mytili*, un pourcentage d'homologie de 15% avec l'ADN de *Vibrio nereis,* et un pourcentage d'homologie de 5% avec l'ADN de *Vibrio tubiashii*.

2. Souche bactérienne selon la revendication 1, possédant les caractéristiques de la souche HE800 déposée le 17 Octobre 1995 auprès de la CNCM sous le numéro I-1629.

3. Souche bactérienne, **caractérisée en ce que** son ADN possède plus que 30% d'homologie avec celui de la souche HE800.

4. Exopolysaccharide susceptible d'être obtenu par précipitation à l'éthanol, à partir d'un surnageant de culture d'une souche bactérienne selon l'une quelconque des revendications 1 à 3, et en ce que :
- il ne comprend pas d'oses neutres ;
- sa teneur en osamines est d'environ 30 ± 5% en poids
- sa teneur en acides uroniques est d'environ 32 ± 5% en poids ;
- sa composition en monosaccharides, déterminée après méthanolyse acide, est la suivante : environ 11,2% en poids d'acide glucuronique, environ 18% en poids de N-acétyl glucosamine, environ 7,9% en poids de N-acétyl galactosamine, soit 1,4 moles de N-acétyl glucosamine et 0,6 moles de N-acétyl galactosamine pour 1 mole d'acide glucuronique ;
- il ne comprend pas d'unités saccharidiques sulfatées ;
- sa viscosité intrinsèque est de l'ordre de 570 ml/g ;
- son poids moléculaire moyen est de l'ordre de 800 000 Da.

5. Exopolysaccharide selon la revendication 4, **caractérisé en ce qu'**il est susceptible d'être obtenu à partir d'un surnageant de culture de la souche HE800 déposée le 17 Octobre 1995 auprès de la CNCM sous le numéro I-1629.

6. Utilisation d'un polysaccharide selon l'une quelconque des revendications 4 ou 5 pour l'obtention d'un médicament.

## Patentansprüche

1. Bakterienstamm der Gattung *Vibrio*, **dadurch** charakterisiert, dass er zur Art mit dem Namen *Vibrio diabolicus* gehört und die folgenden Charakteristika besitzt:
- ein gerades gramnegatives Bazillus, welches ungefähr 0,8 µm breit und 2,2 µm lang ist;
- beweglich mit Hilfe eines polaren Flagellums im flüssigen Kulturmedium und mit Hilfe peritricher Flagellen in festem Kulturmedium;
- keine Kapsel, nicht pigmentiert und nicht luminiszierend;
- fakultativ anaerob, Catalase +, fermentativ, Cytochrom-Oxidase +, Chitinase +, reduzierend Nitrate in Nitrite, und empfindlich gegen 2,4 Diamino-6-7-diisopropylpteridin;
- in der Lage als einzige Kohlenstoffquelle eines der folgenden Substrate zu verwenden: Glycerol, Ribose, Galactose, Glucose, Fructose, Mannose, Mannitol, N-Acetylglucosamin, Maltose, Saccharose, Trehalose, Amidon, Glycogen, Gluconat, Caprat, Citrat, Malat;
- DNA mit einem Gehalt an G+C von 49,6%, einem Homologie-Prozentsatz von 27% mit der DNA von *Vibrio mytili*, einem Homologie-Prozentsatz von 15% mit der DNA von *Vibrio nereis,* und einem Homologie-Prozentsatz von 5% mit der DNA von *Vibrio tubiashii*.

2. Bakterienstamm nach Anspruch 1, welcher die Charakteristika des Stammes HE800 besitzt, hinterlegt am 17. Oktober 1995 bei der CNCM unter der Nummer I-1629.

3. Bakterienstamm, **dadurch gekennzeichnet, dass** seine DNA mehr als 30% Homologie mit der DNA des Stammes HE800 besitzt.

4. Exopolysaccharid, welches mit Hilfe von Ethanol-Präzipitation erhältlich sein kann, ausgehend von einem Kulturüberstand eines Bakterienstamms nach einem der Ansprüche 1 bis 3 und weiterhin **dadurch gekennzeichnet, dass**:
- es keine neutralen Monosaccharide enthält;
- sein Gehalt an Osaminen ungefähr 30 ± 5 Gew.-% ist;
- sein Gehalt an Uronsäuren ungefähr 32 ± 5 Gew.-% ist;
- seine Zusammensetzung an Monosacchariden, bestimmt gemäß saurer Methanolyse, die folgende ist: ungefähr 11,2 Gew.-% an Glucuronsäure, ungefähr 18 Gew.-% an N-Acetylglucosamin, ungefähr 7,9 Gew.-% an N-Acetylgalactosamin, entsprechend 1,4 Mol an N-Acetylglucosamin und 0,6 Mol an N-Acetylgalactosamin pro ein Mol Glucuronsäure;
- es keine sulfatierten Saccharideinheiten umfasst;
- seine intrinsische Viskosität im Bereich von 570 ml/g ist;
- sein mittleres Molekulargewicht im Bereich von 800 000 Da ist.

5. Exopolysaccharid nach Anspruch 4, **dadurch gekennzeichnet, dass** es erhältlich sein kann aus einem Kulturüberstand des Stammes HE800, hinterlegt am 17. Oktober 1995 bei der CNCM unter der Nummer I-1629.

6. Verwendung eines Polysaccharids nach einem der Ansprüche 4 oder 5 zum Erhalt eines Medikaments.

## Claims

1. An isolated bacterial strain of the genus Vibrio, **characterized in that** it belongs to the species called *Vibrio diabolicus*, and possesses the characteristics defined below:
- straight Gram-negative bacillus, about 0.8 µm wide and 2.2 . µm long;
- motile with the aid of a polar flagellum in liquid medium and of peritrichous flagella in solid medium;
- non-encapsulated, non-pigmented and non-luminescent;
- facultatively anaerobic, catalase+, fermentative, cytochrome oxidase+, chitinase+, reducing nitrates to nitrites, and sensitive to 2,4-diamino-6,7-diisopropylpteridine;
- capable of using, as sole carbon source, any one of the following substrates: glycerol, ribose, galactose, glucose, fructose, mannose, mannitol, N-acetylglucosamine, maltose, sucrose, trehalose, starch, glycogen, gluconate, caprate, citrate and malate;
- DNA having a G+C content of 49.6%, a percentage homology of 27% with the DNA of *Vibrio mytili*, a percentage homology of 15% with the DNA of *Vibrio* nereis, and a percentage homology of 5% with the DNA of *Vibrio tubiashii*.

2. The isolated bacterial strain according to claim 1, which possesses the characteristics of the HE800 strain deposited on Oct. 17, 1995 at the CNCM under number I-1629.

3. An isolated bacterial strain, **characterized in that** its DNA possesses more than 30% homology with that of the HE800 strain.

4. An exopolysaccharide obtainable by ethanol precipitation, from the culture supernatant of a bacterial strain according to any one of claims 1 to 3, and wherein:
- the exopolysaccharide does not comprise neutral monosaccharides;
- its content of osamines is about 30.+-.5% by weight;
- its content of uronic acids is about 32.+-.5% by weight;
- its monosaccharide composition, determined after acid methanolysis, is the following: about 11.2% by weight of glucuronic acid, about 18% by weight of N-acetylglucosamine, about 7.9% by weight of N-acetylgalactosamine, that is to say 1.4 moles of N-acetylglucosamine and 0.6 moles of N-acetylgalactosamine per 1 mol of glucuronic acid;
- it does not comprise sulphated saccharide units;
- its intrinsic viscosity is of the order of 570 ml/g;
- its mean molecular weight is of the order of 800,000 Da.

5. The exopolysaccharide according to claim 4 which is obtainable from the culture supernatant of the bacterial strain HE800 strain deposited on Oct. 17, 1995 at the CNCM under No. I-1629.

6. Use of an exopolysaccharide of any one of claims 4 or 5 for the manufacturing of a medicament.
